# EUROPEAN PATENT APPLICATION

(11) **EP 1 862 860 A1**
(43) Date of publication of application: **05.12.2007**
(21) Application number: 06729711.9
(22) Date of filing: 22.03.2006
(51) Int. Cl.: G03G 9/097, C07D 213/81, C07D 309/38

(54) **CHARGE CONTROL AGENT AND RELATED TECHNIQUE**

(30) Priority: 24.03.2005 JP 2005087246
(71) Applicant: ORIENT CHEMICAL INDUSTRIES, LTD., Osaka-shi, Osaka 535-0022 (JP)
(72) Inventor: KURODA, Kazuyoshi c/o ORIENT CHEMICAL IND., LTD., Osaka, 5720813 (JP); YASUMATSU, Masashi c/o ORIENT CHEMICAL IND., LTD., Osaka, 5720813 (JP)
(74) Representative: Liedl, Christine
(86) International application number: PCT/JP2006/305744
(87) International publication number: WO 2006/101144

(57) **Abstract**

[PROBLEMS] Providing a charge control agent that has a negative charge providing property at a practical level, is colorless to light-colored and usable in color toners, produces static charges stable to environmental changes by electrifying the resin powder of a toner and the like, is excellent in storage stability and durability, and is highly safe, as well as a method of controlling the charge of resin powder using the charge control agent, and a toner.

[MEANS OF SOLVING THE PROBLEMS] A charge control agent having a compound represented by the formula shown below as the active ingredient, as well as a method of controlling the charge of resin powder using the charge control agent, and a toner. 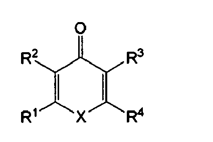 X : an oxygen atom or N-H;
Each of R¹ to R⁴ : a hydrogen atom, a carboxyl group, an aminocarbonyl group having or not having a substituent, an aminocarbonylmethyl group having or not having a substituent, an alkoxycarbonyl group, an alkyl group, a phenyl group having a substituent or not having a substituent, or a group that forms a saturated or unsaturated ring having or not having a substituent in cooperation with any other group selected from among R¹ to R⁴.

## Description

### TECHNICAL FIELD

The present invention relates to a toner for developing electrostatic latent images in electrophotography, electrostatic recording, electrostatic printing and others, a charge control agent capable of controlling the charge amount of the toner etc., a novel compound that functions as the charge control agent, and a method for controlling the charge of a resin powder.

### BACKGROUND ART

In copying machines, printers and other equipment based on electrophotography, various toners containing a coloring agent, a fixing resin and other substances are used to visualize the electrostatic latent image formed on the photoreceptor having a light-sensitive layer containing an inorganic or organic photoconductive substance.

The chargeability of such toners is a particularly important factor in electrostatic latent image developing systems. Thus, to appropriately control or stabilize the charge amount of toner, a charge control agent providing a positive or negative charge is often added to the toner.

Charge control agents providing a negative charge for toner in actual application include metal complex salt dyes of monoazo compounds, and metal complexes or metal salts of aromatic hydroxycarboxylic acids such as alkylsalicylic acids.

Of these, most of metal complexes of azo dye structure that have been proposed as charge control agents are generally poor in stability; for example, they are likely to be decomposed or deteriorated to lose their charge control performance when exposed to mechanical friction or impact, electric impact, light irradiation, temperature or humidity changes, etc. Also, many of these are insufficient in charge stability or unusable in color toners because of chromaticness, even if they have a charge providing property at a practical level. On the other hand, metal complexes of alkyl salicylates and the like are also used in color toners because they are generally light-colored to colorless.

There have been some proposals of charge control agents providing a negative charge which are capable of solving these problems, and contributing to further improvements in the performance of copying machines, printers and the like.

For example, in the official gazette for Japanese Patent Laid-Open No. HEI-10-186728 (patent document 1), metal complexes or metal salts of the following compound are proposed.

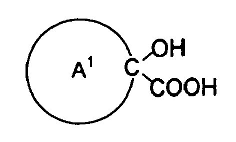

Wherein A¹ represents an organic cyclic residue (for example, fluorene ring, cyclohexyl ring, or xanthene ring).

Also, in the official gazette for Japanese Patent Laid-Open No. HEI-8-50372 (patent document 2), as charge control agents, the following compounds, for example, are disclosed.

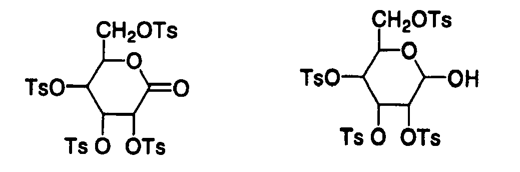

Furthermore, in the official gazette for Japanese Patent Laid-Open No. HEI-10-239910 (patent document 3), negatively chargeable toners comprising a benzene heterocyclic compound such as the following coumarin (derivative), isatoic anhydride (derivative), and isatin (derivative) as a charge control agent are proposed.

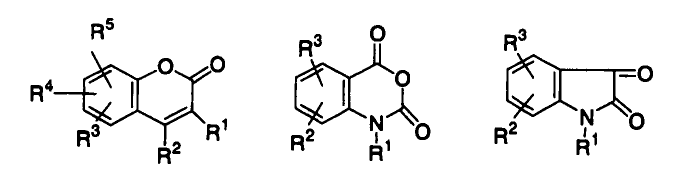

Also, in the official gazette for Japanese Patent Laid-Open No. HEI-4-139461 (patent document 4), as charge control agents, the following compounds, for example, are disclosed.

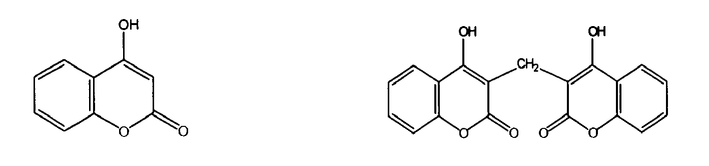

However, as is evident in Comparative Examples below, an investigation of the characteristics of the charge control agents described in the aforementioned official gazettes for Japanese Patent Laid-Open No. HEI-10-186728, Japanese Patent Laid-Open No. HEI-8-50372, Japanese Patent Laid-Open No. HEI-10-239910, and Japanese Patent Laid-Open No. HEI-4-139461 showed that in all these cases, the charge control agents are colorless to light-colored but do not offer sufficient charge amounts or environmental stability.
[Patent document 1] Official gazette for Japanese Patent Laid-Open No. HEI-10-186728
[Patent document 2] Official gazette for Japanese Patent Laid-Open No. HEI-8-50372
[Patent document 3] Official gazette for Japanese Patent Laid-Open No. HEI-10-239910
[Patent document 4] Official gazette for Japanese Patent Laid-Open No. HEI-4-139461

### DISCLOSURE OF THE INVENTION

### PROBLEMS TO BE SOLVED BY THE INVENTION

The present invention was developed in view of the aforementioned problems in the prior art and is intended to provide:
a charge control agent that:
   (1) has a negative charge providing property at a practical level,
   (2) is colorless to light-colored and can be used in color toners,
   (3) produces static charges, by electrification of the resin powder of the toner and the like used, stable to environmental changes,
   (4) is excellent in storage stability [charge control characteristic stability over time],
   (5) is excellent in durability (charge control characteristic stability of the toner and the like used in multiple repeated use), and
   (6) does not contain harmful heavy metals and is highly safe,
a novel compound that functions as the charge control agent, a method of controlling the charge of resin powder using the charge control agent, and
a toner for developing electrostatic images which offers good fixability in a broad range of temperature and offset resistance, which is excellent in environmental resistance (charge characteristic stability to temperature and
humidity changes), storage stability (charge characteristic stability over time) and durability (charge characteristic stability of toner in multiple repeated use), and which produces stable toner images.

### MEANS FOR SOLVING THE PROBLEMS

(1-1) Accomplishing the above-described objects, the charge control agent of the present invention has a compound comprising a 4-pyrone ring or a 4-1H-pyridone ring, represented by the following General Formula (I), as the active ingredient:

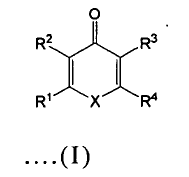

in Formula (I),
X represents an oxygen atom or N-H,
each of R¹ to R⁴ independently represents:
a hydrogen atom,
a carboxyl group,
an aminocarbonyl group having or not having a substituent,
an aminocarbonylmethyl group having or not having a substituent,
an alkoxycarbonyl group,
an alkyl group,
a phenyl group having a substituent or not having a substituent,
a naphthyl group having a substituent or not having a substituent, or
a group that forms a saturated or unsaturated ring having or not having a substituent in cooperation with any other group selected from among R¹ to R⁴.

(1-2) The charge control agent of the present invention is preferably one wherein the compound of General Formula (I) above is 1 or 2 or more selected from among the following compounds 1 to 6:

Compound 1

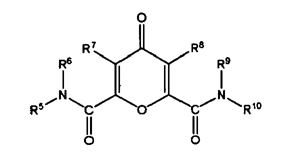

Compound 2

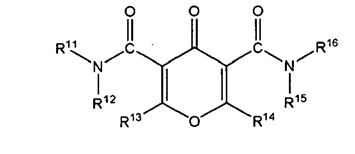

Compound 3

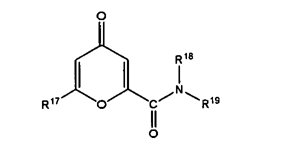

Compound 4

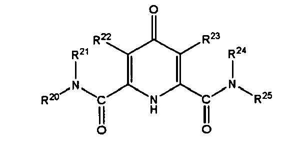

Compound 5

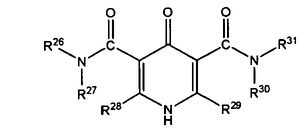

Compound 6

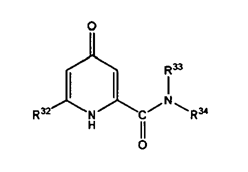

in Compounds 1 to 6, each of R⁵ to R³⁴ independently represents a hydrogen atom, an alkyl group, a cycloalkyl group not having a substituent or having a substituent, a phenyl group having a substituent or not having a substituent, or a naphthyl group having a substituent or not having a substituent. As examples of the substituent in these phenyl group and naphthyl group, alkyl groups, cycloalkyl groups, halogen atoms, sulfonic acid ester groups, aryl groups, acylamino groups, arylcarbonylamino groups, aminocarbonyl groups, alkoxy groups, acyl groups, and arylcarbonyl groups can be mentioned. The aforementioned phenyl group and naphthyl group may have 1 or 2 or more of these substituents on the aromatic ring thereof. Provided that 2 or more substituents are present, they may be mutually identical or different.

(1-3) Also, the charge control agent of the present invention is preferably one wherein the compound of General Formula (I) above is one or both of the following compounds 7 and 8:

Compound 7

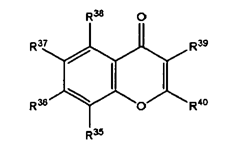

Compound 8

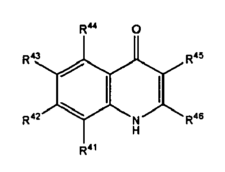

in Compounds 7 and 8, each of R³⁵ to R⁴⁶ independently represents a hydrogen atom, an alkyl group, a phenyl group having a substituent or not having a substituent, a naphthyl group having a substituent or not having a substituent, an aminocarbonyl group having or not having a substituent, or a carboxyl group. As examples of the substituent in the aforementioned phenyl group and naphthyl group, alkyl groups, cycloalkyl groups, halogen atoms, sulfonic acid ester groups, aryl groups, acylamino groups, arylcarbonylamino groups, aminocarbonyl groups, alkoxy groups, acyl groups, and arylcarbonyl groups can be mentioned. The aforementioned phenyl group and naphthyl group may have 1 or 2 or more of these substituents on the aromatic ring thereof. Provided that 2 or more substituents are present, they may be mutually identical or different.

(1-4) Also, the charge control agent of the present invention is preferably one wherein the compound of General Formula (I) above is one or both of the following compounds 9 and 10:

Compound 9

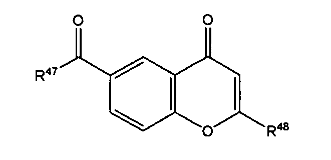

Compound 10

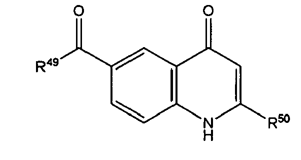

in Compounds 9 and 10,
each of R⁴⁷ and R⁴⁹ independently represents a hydroxyl group or an amino group having a substituent or not having a substituent,
each of R⁴⁸ and R⁵⁰ independently represents a hydrogen atom, an alkyl group, a phenyl group having a substituent or not having a substituent, or a naphthyl group having a substituent or not having a substituent. As examples of the substituent in these phenyl group and naphthyl group, alkyl groups, cycloalkyl groups, halogen atoms, sulfonic acid ester groups, aryl groups, acylamino groups, arylcarbonylamino groups, aminocarbonyl groups, alkoxy groups, acyl groups, and arylcarbonyl groups can be mentioned. The aforementioned phenyl group and naphthyl group may have 1 or 2 or more of these substituents on the aromatic ring thereof. Provided that 2 or more substituents are present, they may be mutually identical or different.

(2) The toner of the present invention for developing electrostatic images comprises a resin for toner, a coloring agent, and any of the above-described charge control agents.

(3) The charge control method of the present invention is to control the charge of a resin powder by containing any of the above-described charge control agents in the resin powder.

(4) The compound of the present invention is a compound represented by the following Formula (II) or a compound represented by the following Formula (III):

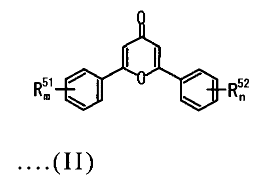

in Formula (II),
each of R⁵¹ and R⁵² independently represents an unbranched or branched alkyl group having 1 to 18 carbon atoms, each of m and n independently represents an integer of 0 to 3.
Note that the alkyl groups mentioned herein are not subject to limitation regarding the presence or absence of branching unless otherwise stated:

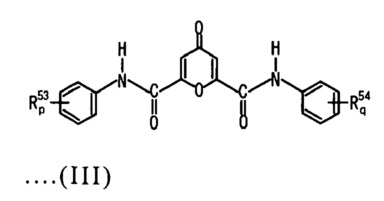

in Formula (III),
each of R⁵³ and R⁵⁴ independently represents an unbranched or branched alkyl group having 1 to 18 carbon atoms, each of p and q independently represents an integer of 0 to 3.

### EFFECT OF THE INVENTION

The charge control agent and novel compound of the present invention are excellent in negative charge providing property and its stability, good in dispersibility in resins for toner, and excellent in the environmental stability of charge amount when used in toners, excellent in storage stability and durability, highly safe because it does not contain harmful heavy metals, and because it is colorless or light-colored, it is unlikely to cause color tone damage when used in toners and the like.

According to the charge control method of the present invention, control of the negative charge of resin powder can be achieved stably, and because it uses a colorless to light-colored charge control agent that does not contain harmful heavy metals, it is highly safe and it is unlikely to cause color tone damage on resin powder.

The toner of the present invention for developing electrostatic images offers good fixability and non-offset property in a broad range of temperature, is excellent in environmental resistance, storage stability and durability, and is capable of forming stable copied images.

### BEST MODE FOR EMBODYING THE INVENTION

The charge control agent of the present invention has a compound comprising a 4-pyrone ring or a 4-1H-pyridone ring, represented by General Formula (I) above, as the active ingredient.

Regarding an aminocarbonyl group having or not having a substituent, an aminocarbonylmethyl group having or not having a substituent, an alkoxycarbonyl group, and an alkyl group for R¹ to R⁴ in General Formula (I) above, the following examples can be mentioned, respectively. However, these are not to be construed as limiting the scope of the present invention.

As the aminocarbonyl group, carbamoyl groups and N- or N,N-substituted carbamoyl groups can be mentioned; as examples of the (1 or 2) substituent(s) in the latter, alkyl groups having 1 to 18 carbon atoms (preferably 1 to 8 carbon atoms), cycloalkyl groups not having a substituent or having a substituent, and phenyl groups or naphthyl groups not having a substituent or having [an alkyl group having 1 to 18 carbon atoms (preferably 1 to 8 carbon atoms), a cycloalkyl group, a halogen atom, a sulfonic acid ester group, an aryl group, an acylamino group, an arylcarbonylamino group, an aminocarbonyl group, an alkoxy group, an acyl group, an arylcarbonyl group and the like] as a substituent can be mentioned. As more specific examples of the N- or N,N-substituted carbamoyl groups, N-methylaminocarbonyl groups, N-t-butylaminocarbonyl groups, N-phenylaminocarbonyl groups, N-(4-t-butylphenyl)aminocarbonyl groups, N,N-diphenylaminocarbonyl groups, N-(4-t-butylcyclohexyl)aminocarbonyl groups, N-(2-chlorophenyl)aminocarbonyl groups, N-[2-(phenoxysulfonyl)phenyl]aminocarbonyl groups, N-(4-biphenylyl)aminocarbonyl groups, N-{4-[(t-butylamino)carbonyl]phenyl}aminocarbonyl groups, N-[4-(t-butylcarbonylamino)phenyl]aminocarbonyl groups, N-(4-t-butoxyphenyl)aminocarbonyl groups, N-[4-(t-butylcarbonyl)phenyl]aminocarbonyl groups and the like, N-(2-naphthyl)aminocarbonyl groups, N-(4-t-butyl-1-naphthyl)aminocarbonyl groups, N,N-di(2-naphthyl)aminocarbonyl groups, N-(4-chloro-2-naphthyl)aminocarbonyl groups, N-[2-(phenoxysulfonyl)-1-naphthyl]aminocarbonyl groups, N-{4-[(t-butylamino)carbonyl]-2-naphthyl}aminocarbonyl groups, N-[4-(t-butylcarbonylamino)-1-naphthyl]aminocarbonyl groups, N-(4-t-butoxy-2-naphthyl)aminocarbonyl groups, and N-[4-(t-butylcarbonyl)-1-naphthyl]aminocarbonyl groups can be mentioned.

As the aminocarbonylmethyl group, carbamoyl methyl groups and Nor N,N-substituted carbamoyl methyl groups can be mentioned; as examples of the (1 or 2) substituent(s) in the latter, alkyl groups having 1 to 18 carbon atoms (preferably 1 to 8 carbon atoms), cycloalkyl groups not having a substituent or having a substituent, and phenyl groups or naphthyl groups not having a substituent or having [an alkyl group having 1 to 18 carbon atoms (preferably 1 to 8 carbon atoms), a cycloalkyl group, a halogen atom, a sulfonic acid ester group, an aryl group, an acylamino group, an arylcarbonylamino group, an aminocarbonyl group, an alkoxy group, an acyl group, an arylcarbonyl group and the like] as a substituent can be mentioned. As more specific examples of the N- or N,N-substituted carbamoyl methyl groups, N-methylaminocarbonylmethyl groups, N-t-butylaminocarbonylmethyl groups, N-phenylaminocarbonylmethyl groups, N-(4-t-butylphenyl)aminocarbonylmethyl groups, N,N-diphenylaminocarbonylmethyl groups, N-(4-t-butylcyclohexyl)aminocarbonylmethyl groups, N-(2-chlorophenyl)aminocarbonylmethyl groups, N-[2-(phenoxysulfonylphenyl)aminocarbonylmethyl groups, N-(4-biphenylyl)aminocarbonylmethyl groups, N-{4-[(t-butylamino)carbonyl]phenyl}aminocarbonylmethyl groups, N-[4-(t-butylcarbonylamino)phenyl]aminocarbonylmethyl groups, N-(4-t-butoxyphenyl)aminocarbonylmethyl groups, N-[4-(t-butylcarbonyl)phenyl]aminocarbonylmethyl groups, N-(2-naphthyl)aminocarbonylmethyl groups, N-(4-t-butyl-1-naphthyl)aminocarbonylmethyl groups, N,N-di(2-naphthyl)aminocarbonylmethyl groups, N-(4-chloro-2-naphthyl)aminocarbonylmethyl groups, N-[2-(phenoxysulfonyl)-1-naphthyl]aminocarbonylmethyl groups, N-{4-[(t-butylamino)carbonyl]-2-naphthyl}aminocarbonylmethyl groups, N-[4-(t-butylcarbonylamino)-1-naphthyl]aminocarbonylmethyl groups, N-(4-t-butoxy-2-naphthyl)aminocarbonylmethyl groups, N-[4-(t-butylcarbonyl)-1-naphthyl]aminocarbonylmethyl groups and the like can be mentioned.

As examples of the alkoxycarbonyl group, alkoxycarbonyl groups wherein the alkoxy group has 1 to 18 carbon atoms (preferably 1 to 8 carbon atoms), such as methoxycarbonyl groups, ethoxycarbonyl groups, and t-butoxycarbonyl groups, can be mentioned.

As examples of the alkyl group, alkyl groups having 1 to 18 carbon atoms (preferably 1 to 8 carbon atoms), such as methyl groups, ethyl groups, propyl groups, isopropyl groups, butyl groups, and t-butyl groups, can be mentioned.

As the substituent in the phenyl group having a substituent and the naphthyl group having a substituent for R¹ to R⁴, alkyl groups having 1 to 18 carbon atoms, cycloalkyl groups, halogen atoms, sulfonic acid ester groups, aryl groups, acylamino groups, arylcarbonylamino groups, aminocarbonyl groups, alkoxy groups, acyl groups, and arylcarbonyl groups are preferable. The aforementioned phenyl group or naphthyl group may have 1 or 2 or more of these substituents on the aromatic ring thereof. Provided that 2 or more substituents are present, they may be mutually identical or different.

More specific examples of the respective substituents are given below, but are not to be construed as limiting the scope of the invention.
As examples of the alkyl groups, methyl groups, ethyl groups, propyl groups, isopropyl groups, butyl groups, t-butyl groups and the like can be mentioned.

As examples of the cycloalkyl groups, cyclopentyl groups, cyclohexyl groups, cycloheptyl groups and the like can be mentioned.

As examples of the halogen atoms, fluorine atoms, chlorine atoms, bromine atoms, iodine atoms and the like can be mentioned.

As examples of the sulfonic acid ester groups, methyl sulfonate, ethyl sulfonate, butyl sulfonate groups, phenyl sulfonate groups and the like can be mentioned.

As examples of the aryl groups, phenyl groups, tolyl groups, naphthyl groups and the like can be mentioned.

As examples of the aminocarbonyl groups, carbamoyl groups, N-methylaminocarbonyl groups, N-(4-t-butylphenyl)aminocarbonyl groups and the like can be mentioned.

As examples of the acylamino groups, acetylamino groups, ethylcarbonylamino groups, t-butylcarbonylamino groups and the like can be mentioned.

As examples of the arylcarbonylamino groups, phenylcarbonylamino groups, (4-t-butylphenyl)carbonylamino groups, 1-naphthylcarbonylamino groups and the like can be mentioned.

As examples of the alkoxy groups, methoxy groups, ethoxy groups, t-butoxy groups and the like can be mentioned.

As examples of the acyl groups, acetyl groups, ethylcarbonyl, t-butylcarbonyl groups and the like can be mentioned. As examples of the arylcarbonyl groups, phenylcarbonyl groups, (4-t-butylphenyl)carbonyl groups, 1-naphthylcarbonyl groups and the like can be mentioned.

Also, provided that one of R¹ to R⁴ forms a saturated or unsaturated ring having or not having a substituent in cooperation with any other group selected from among R¹ to R⁴, that is, provided that two groups optionally chosen from among R¹ to R⁴ in General Formula (I) (particularly, R¹ and R², R³ and R⁴) form a saturated or unsaturated ring having or not having a substituent, as examples of the saturated or unsaturated ring, saturated or unsaturated carbon or heterocyclic (for example, heterocyclic ring consisting of carbon and nitrogen or oxygen) 3- to 8-membered rings such as cyclopropene ring, cyclopropane ring, cyclobutadiene ring, cyclobutane ring, cyclopentadiene ring, cyclopentane ring, cyclohexane ring, cycloheptatriene ring, benzene ring, pyridine ring, and pyrane ring can be mentioned. However, these are not to be construed as limiting the scope of the present invention.

As examples of the substituent present in this saturated or unsaturated ring, alkyl groups having 1 to 18 carbon atoms (preferably 1 to 8 carbon atoms), such as methyl groups, ethyl groups, propyl groups, isopropyl groups, butyl groups, and t-butyl groups, carboxyl groups, carbamoyl groups, N- or N,N-substituted carbamoyl groups and the like can be mentioned. As examples of the (1 or 2) substituent(s) in the N- or N,N-substituted carbamoyl groups, alkyl groups having 1 to 18 carbon atoms (preferably 1 to 8 carbon atoms), cycloalkyl groups not having a substituent or having a substituent, phenyl groups or naphthyl groups not having a substituent or having [an alkyl group having 1 to 18 carbon atoms (preferably 1 to 8 carbon atoms), a cycloalkyl group, a halogen atom, a sulfonic acid ester group, an aryl group, an acylamino group, an arylcarbonylamino group, an aminocarbonyl group, an alkoxy group, an acyl group, an arylcarbonyl group and the like] as a substituent and the like can be mentioned. As more specific examples of the N- or N,N-substituted carbamoyl groups, N-methylaminocarbonyl groups, N-t-butylaminocarbonyl groups, N-phenylaminocarbonyl groups, N-(4-t-butylphenyl)aminocarbonyl groups, N,N-diphenylaminocarbonyl groups, N-(4-t-butylcyclohexyl)aminocarbonyl groups, N-(2-chlorophenyl)aminocarbonyl groups, N-[2-(phenoxysulfonyl)phenyl]aminocarbonyl groups, N-(4-biphenylyl)aminocarbonyl groups, N-{4-[(t-butylamino)carbonyl]phenyl}aminocarbonyl groups, N-[4-(acetylamino)phenyl]aminocarbonyl groups, N-(4-t-butoxyphenyl)aminocarbonyl groups, N-[4-(t-butylcarbonyl)phenyl]aminocarbonyl groups, N-(2-naphthyl)aminocarbonyl groups, N-(4-t-butyl-1-naphthyl)aminocarbonyl groups, N,N-di(2-naphthyl)aminocarbonyl groups, N-(4-chloro-2-naphthyl)aminocarbonyl groups, N-[2-(phenoxysulfonyl)-1-naphthyl]aminocarbonyl groups, N-{4-[(t-butylamino)carbonyl]-2-naphthyl}aminocarbonyl groups, N-[4-(t-butylcarbonylamino)-1-naphthyl]-amino-carbonyl groups, N-(4-t-butoxy-2-naphthyl)aminocarbonyl groups, N-[4-(t-butylcarbonyl)-1-naphthyl]aminocarbonyl groups and the like can be mentioned.

Examples of the alkyl group for R⁵ to **R⁴⁶,** R⁴⁸, and R⁵⁰, examples of the substituent in the phenyl group having a substituent, and examples of the aminocarbonyl group for R³⁵ to R⁴⁶ in the above-described compounds 1 to 10 are the same as those for the aforementioned R¹ to R⁴.

As the amino group that may have a substituent for R⁴⁷ and R⁴⁹ in the above-described compounds 1 to 10, N- or N,N-substituted or unsubstituted amino groups can be mentioned. As the (1 or 2) substituent(s), alkyl groups having 1 to 18 carbon atoms (preferably 1 to 8 carbon atoms) and phenyl groups or naphthyl groups not having a substituent or having [an alkyl group having 1 to 18 carbon atoms, preferably 1 to 8 carbon atoms, a cycloalkyl group, a halogen atom, a sulfonic acid ester group, an aryl group, an acylamino group, an arylcarbonylamino group, an aminocarbonyl group, an alkoxy group, an acyl group, an arylcarbonyl group and the like] as a substituent can be mentioned. As more specific examples of the Nor N,N-substituted or unsubstituted amino groups, amino groups, N-methylamino groups, N-t-butylamino groups, N-phenylamino groups, N-(4-t-butylphenyl)amino groups, N,N-diphenylamino groups, N-(4-t-butylcyclohexyl)amino groups, N-(2-chlorophenyl)amino groups, N-[2-(phenoxysulfonyl)phenyl]amino groups, N-(4-biphenylyl)amino groups, N-{4-[(t-butylamino)carbonyl]phenyl}amino groups, N-[4-(acetylamino)phenyl]amino groups, N-(4-t-butoxyphenyl)amino groups, N-[4-(t-butylcarbonyl)phenyl]amino groups, N-(2-naphthyl)aminocarbonyl groups, N-(4-t-butyl-1-naphthyl)aminocarbonyl groups, N,N-di(2-naphthyl)aminocarbonyl groups, N-(4-chloro-2-naphthyl)aminocarbonyl groups, N-[2-(phenoxysulfonyl)-1-naphthyl]aminocarbonyl groups, N-{4-[(t-butylamino)carbonyl]-2-naphthyl}aminocarbonyl groups, N-[4-(t-butylcarbonylamino)-1-naphthyl]-aminocarbonyl groups, N-(4-t-butoxy-2-naphthyl)aminocarbonyl groups, N-[4-(t-butylcarbonyl)-1-naphthyl]aminocarbonyl groups and the like can be mentioned.

Note that the compound comprising a 4-pyrone ring or a 4-1H-pyridone ring, represented by General Formula (I), is preferably a compound wherein each of R¹ to R⁴ is:
a hydrogen atom;
an alkyl group having 1 to 18 (preferably 1 to 8) carbon atoms;
an aminocarbonyl group not having a substituent or N- or N,N-substituted [as examples of the (1 or 2) substituent(s), alkyl groups having 1 to 18 carbon atoms (preferably 1 to 8 carbon atoms), cycloalkyl groups not having a substituent or having a substituent, and phenyl groups or naphthyl groups not having a substituent or having a substituent can be mentioned; as examples of the substituent for the phenyl groups and naphthyl groups, alkyl groups having 1 to 18 carbon atoms (preferably 1 to 8 carbon atoms), cycloalkyl groups, halogen atoms, sulfonic acid ester groups, aryl groups, acylamino groups, arylcarbonylamino groups, aminocarbonyl groups, alkoxy groups, acyl groups, arylcarbonyl groups and the like can be mentioned.];
a phenyl group or naphthyl group not having a substituent or having a substituent [as examples of the substituent, alkyl groups having 1 to 18 (preferably 1 to 8) carbon atoms, cycloalkyl groups, halogen atoms, sulfonic acid ester groups, aryl groups, acylamino groups, arylcarbonylamino groups, aminocarbonyl groups, alkoxy groups, acyl groups, arylcarbonyl groups and the like can be mentioned.];
or
a 6-membered ring not having a substituent or having a substituent, formed by two groups [as examples of the substituent, alkyl groups having 1 to 18 carbon atoms (preferably 1 to 8 carbon atoms), carboxyl groups, or N- or N,N-substituted aminocarbonyl groups can be mentioned; as the (1 or 2) substituent(s) for the N- or N,N-substituted aminocarbonyl groups, phenyl groups or naphthyl groups not having a substituent or having a substituent can be mentioned; as examples of the substituent, alkyl groups having 1 to 18 carbon atoms (preferably 1 to 8 carbon atoms), cycloalkyl groups, halogen atoms, sulfonic acid ester groups, aryl groups, acylamino groups, arylcarbonylamino groups, aminocarbonyl groups, alkoxy groups, acyl groups, arylcarbonyl groups and the like can be mentioned.]. In this case as well, some or all of R¹ to R⁴ may be mutually different or identical.

As specific preferable examples of the compound comprising a 4-pyrone ring or a 4-1H-pyridone ring, represented by General Formula (I), the following Example Compounds 1 to 43 can be mentioned. However, preferable examples are not limited thereto.

### [Example Compound 1]

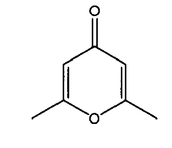

### [Example Compound 2]

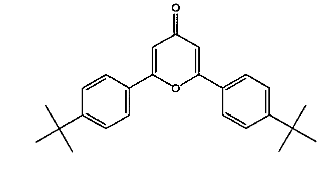

### [Example Compound 3]

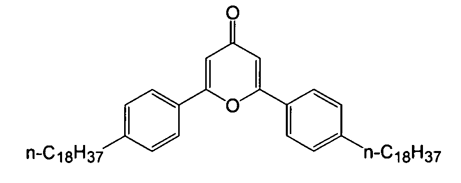

### [Example Compound 4]

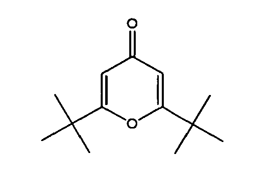

### [Example Compound 5]

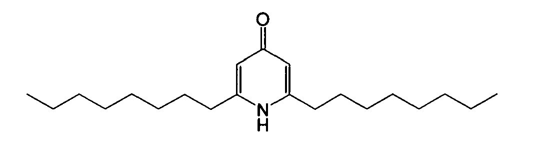

### [Example Compound 6]

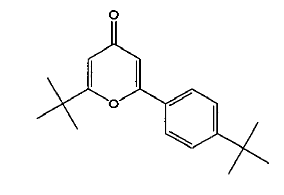

### [Example Compound 7]

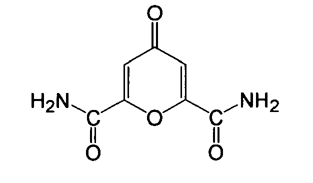

### [Example Compound 8]

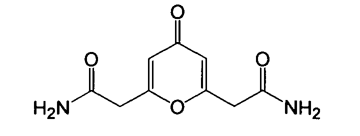

### [Example Compound 9]

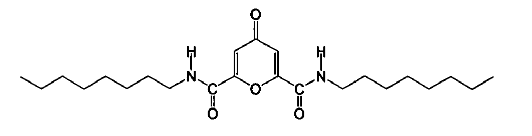

### [Example Compound 10]

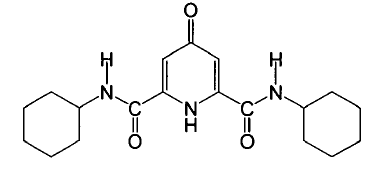

### [Example Compound 11]

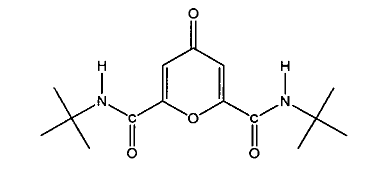

### [Example Compound 12]

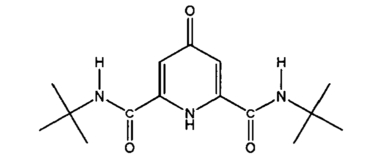

### [Example Compound 13]

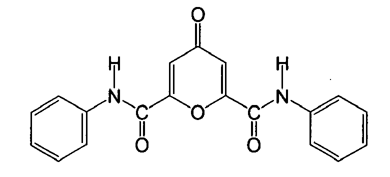

### [Example Compound 14]

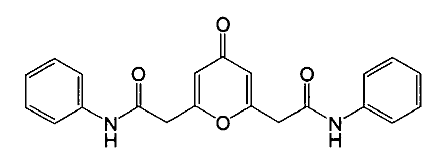

### [Example Compound 15]

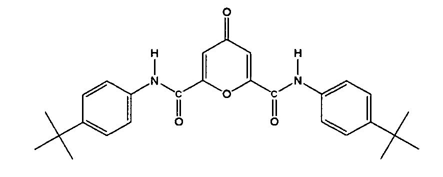

### [Example Compound 16]

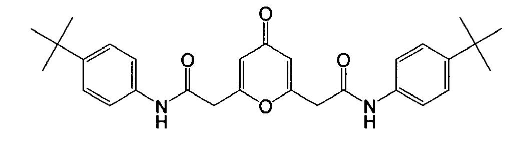

### [Example Compound 17]

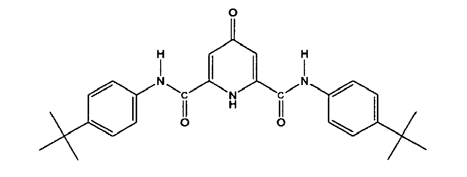

### [Example Compound 18]

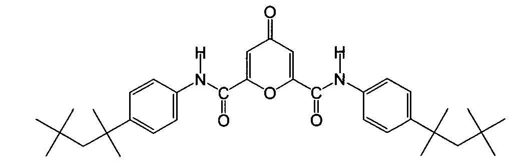

### [Example Compound 19]

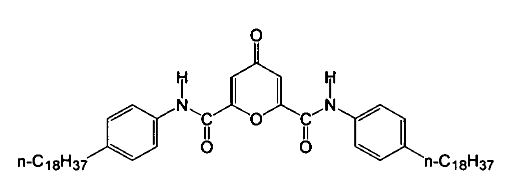

### [Example Compound 20]

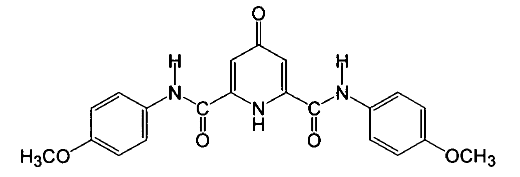

### [Example Compound 21]

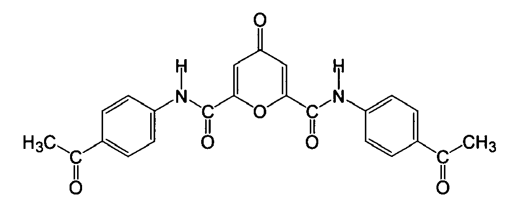

### [Example Compound 22]

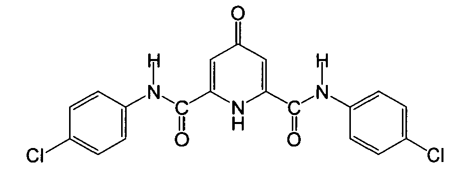

### [Example Compound 23]

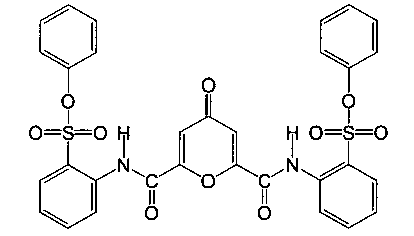

### [Example Compound 24]

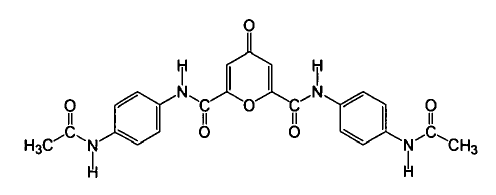

### [Example Compound 25]

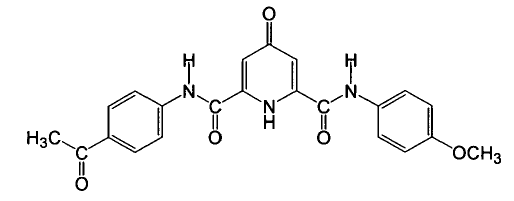

### [Example Compound 26]

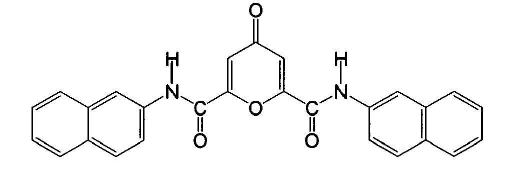

### [Example Compound 27]

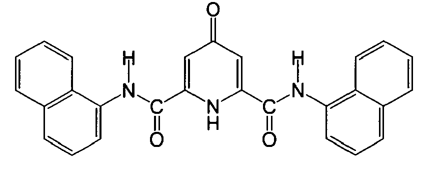

### [Example Compound 28]

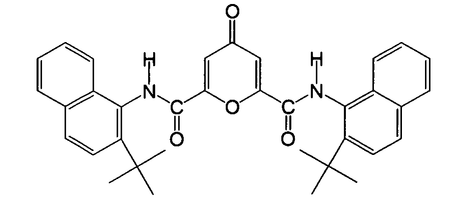

### [Example Compound 29]

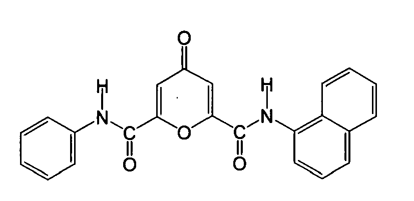

### [Example Compound 30]

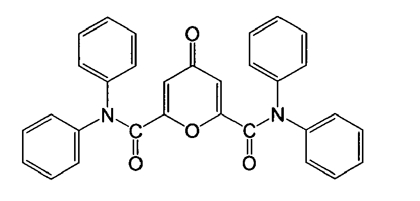

### [Example Compound 31]

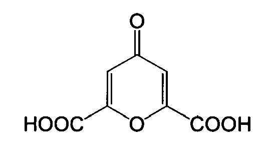

### [Example Compound 32]

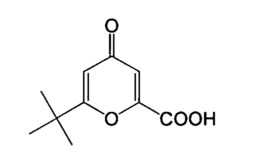

### [Example Compound 33]

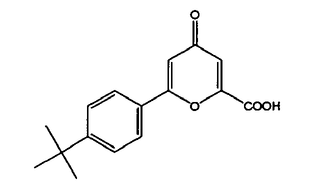

### [Example Compound 34]

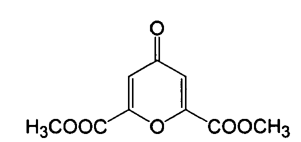

### [Example Compound 35]

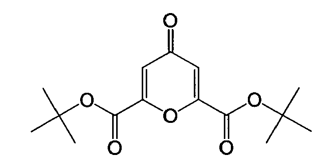

### [Example Compound 36]

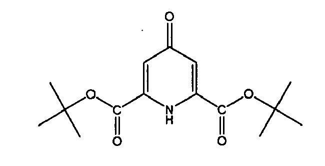

### [Example Compound 37]

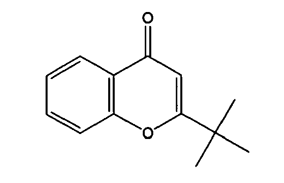

### [Example Compound 38]

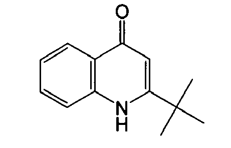

### [Example Compound 39]

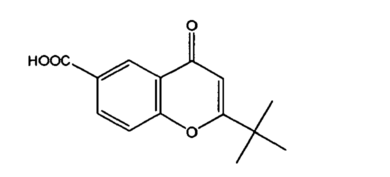

### [Example Compound 40]

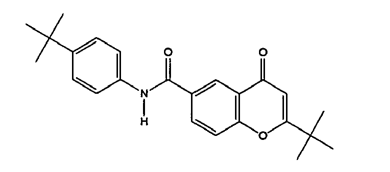

### [Example Compound 41]

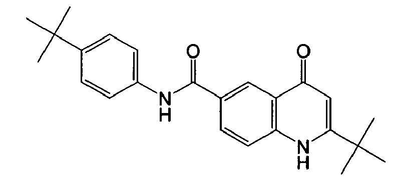

### [Example Compound 42]

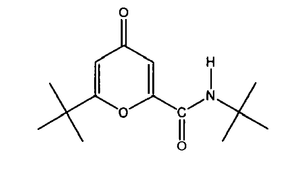

### [Example Compound 43]

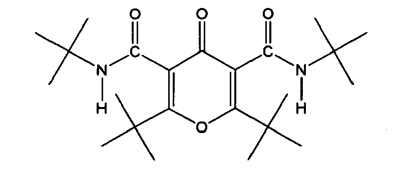

The above-described charge control agent of the present invention preferably has a particle diameter of not more than 10 µm. The particle diameter is more preferably not more than 3 µm, still more preferably not more than 1 µ m. Although there is no lower limit of particle diameter, the particle diameter may, for example, be not less than 0.01 µ m. A charge control agent having a desired particle diameter can be obtained by dry or wet pulverization using various milling machines such as a ball mill and bead mill, or by recrystallization, re-precipitation and the like.
Also, the charge control agent of the present invention preferably has a low ionic component content. Specifically, at the time of water washing, the electroconductivity of the filtrate is preferably not more than 500 µ Scm⁻¹, more preferably not more than 300 µ Scm⁻¹.

Also, the charge control agent of the present invention preferably has a purity (percentage by weight of the compound represented by General Formula (I) in the charge control agent) of not less than 90%, more preferably not less than 95%.
Next, the toner of the present invention for developing electrostatic images comprises a resin for toner, a coloring agent, and the above-described charge control agent of the present invention. The compound represented by General Formula (I), which constitutes the active ingredient of the charge control agent of the present invention in the toner may be a single kind of compound, or may be a mixture of several kinds of compounds. Also, the toner of the present invention for developing electrostatic images may comprise another charge control agent (for example, azo metal complexes, salicylic acid metal complexes and the like).

The toner of the present invention for developing electrostatic images is desirably formulated with a compound comprising a 4-pyrone ring or a 4-1H-pyridone ring, represented by General Formula (I) above, at 0.1 to 10 parts by weight to 100 parts by weight of the resin for toner. A more preferable amount of charge control agent formulated is 0.5 to 5 parts by weight to 100 parts by weight of the resin for toner.

As examples of the resin for toner that can be used in the toner of the present invention for developing electrostatic images, the following known resins for toner (binder resins) can be mentioned. Specifically, thermoplastic resins such as styrene resin, styrene-acrylic resin, styrenebutadiene resin, styrene-maleic resin, styrene-vinyl methyl ether resin, styrene-methacrylic acid ester copolymer, polyester resin, and polypropylene resin can be mentioned. These resins can also be used singly or in blends of several kinds. Note that the charge control agent of the present invention can also be contained in an electrostatic powder paint and used to control (enhance) the charge of a resin powder. In this case, as examples of the resin for paint, thermoplastic resins such as those of the acrylic series, polyolefin series, polyester series, or polyamide series, and thermosetting resins such as those of the phenol series, epoxy series, and polyester series can be mentioned, and these can be used singly or in blends of several kinds.

The toner of the present invention may incorporate various dyes and pigments as coloring agents, singly or in combination of 2 kinds or more. Examples of such dyes and pigments are as follows. Namely, organic pigments such as Quinophthalone Yellow, Isoindolinone Yellow, Benzidine Yellow, Perinone Orange, Perinone Red, Perirene Maroon, Rhodamine 6G Lake, Quinacridone Red, Anthanthrone Red, Rose Bengale, copper Phthalocyanine Blue, copper Phthalocyanine Green, and diketopyrrolopyrrole pigments; inorganic pigments and metal powders such as Carbon Black, Titanium White, Titanium Yellow, Ultramarine, Cobalt Blue, red iron oxide, aluminum powder, and bronze; various oil-soluble dyes and disperse dyes such as azo dyes, quinophthalone dyes, anthraquinone dyes, phthalocyanine dyes, indophenol dyes, and indoaniline dyes; and triarylmethane dyes and xanthene dyes modified by resins such as rosin, rosin-modified phenol, and rosin-modified maleic acid, and the like.

The toner of the present invention for developing electrostatic images is produced, for example, as described below.

A toner having an average particle diameter of 5 to 20 µ m can be obtained by thoroughly mixing a resin for toner and coloring agent as described above, and the charge control agent of the present invention, and, if necessary, a magnetic material (for example, fine powders of ferromagnetic materials such as iron, cobalt, and ferrite), a fluidizing agent (for example, silica, aluminum oxide, titanium oxide), an anti-offset agent (for example, waxes, low molecular olefin waxes) and the like, using a ball mill or another mechanical mixer, subsequently kneading the mixture in a molten state using a hot kneader such as a hot roll, a kneader or an extruder, cooling, solidifying and then pulverizing the mixture, and classifying the particles.

Also, a method comprising dispersing the starting materials in a binder resin solution and then spray-drying the solution to obtain the desired product, or the polymerizing toner production method comprising mixing a given set of starting materials in a monomer for the binder resin to yield an emulsified suspension which is then polymerized to yield a toner (what is called polymerized toner) and the like can be applied.

For example, in the suspension polymerization method, by uniformly dissolving or dispersing a polymerizable monomer, a coloring agent and a charge control agent, and, if necessary, a polymerization initiator, a crosslinking agent, a releasing agent, and other additives to obtain a monomer composition, then dispersing this monomer composition in a continuous layer (for example, aqueous phase) containing a dispersion stabilizer using an appropriate dispersing machine, and allowing a polymerization reaction to proceed, toner particles having a desired particle diameter can be obtained.

As examples of the polymerizable monomer for forming a resin for polymerized toner, styrene derivatives such as styrene, methylstyrene; (meth)acrylic acid esters such as methyl acrylate, ethyl acrylate, ethyl methacrylate, and n-butyl methacrylate; and vinyl-series monomers such as acrylonitrile, methacrylonitrile, and acrylamide can be mentioned.

As the aforementioned dispersion stabilizer, various surfactants (for example, sodium dodecylbenzenesulfonate), organic or inorganic dispersing agents and the like can be used. As examples of the organic dispersing agents, polyvinyl alcohol, methylcellulose, methylhydroxypropylcellulose and the like can be mentioned. As examples of the inorganic dispersing agents, fine powders of polyvalent metal salts of phosphoric acid such as calcium phosphate, magnesium phosphate, and aluminum phosphate; fine powders of carbonates such as calcium carbonate and magnesium carbonate; inorganic dispersing agents such as calcium metasilicate, calcium sulfate, barium sulfate, calcium hydroxide, and aluminum hydroxide and the like can be mentioned.

As examples of the aforementioned polymerization initiator, azo-series or diazo-series polymerization initiators such as 2,2'-azoisobutyronitrile and azobisbutyronitrile; peroxide-series polymerization initiators such as benzoyl peroxide and the like can be mentioned.

When the toner of the present invention is used as a two-component developer, the toner of the present invention can be mixed with carrier powder and development can be achieved by the magnetic brush developing process or the like.

The carrier is not subject to limitation; any known carrier can be used. Examples of the carrier include iron powder, nickel powder, ferrite powder and glass beads about 50 to 200 µm in particle diameter, and such materials as coated with acrylate copolymer, styrene-acrylate copolymer, styrene-methacrylate copolymer, silicone resin, polyamide resin, ethylene fluoride resin or the like.

When the toner of the present invention is used as a one-component developer, an appropriate amount of a fine powder of a ferromagnetic material such as iron powder, nickel powder or ferrite powder may be added and dispersed in preparing the toner as described above. Examples of developing processes that can be used in this case include contact development and jumping development.

In the development of electrostatic images using the toner of the present invention described above, the charge of the toner is controlled by the charge control agent of the present invention in the toner.

### EXAMPLES

The toner of the present invention is hereinafter described in more detail by means of the following examples, but these are not to be construed as limitative on the present invention. In the description below, "part(s) by weight" are referred to as "part(s)" for short.

### Example 1 (Synthesis of Example Compound 2)

To a solution of 10.0 g (52.0 mmol) of methyl 4-t-butylbenzoate dissolved in 200 mL of 1,2-dimethoxyethane, 3.37 g (62.4 mmol) of sodium methoxide was added, and the solution was refluxed. A solution of 3.02 g (52.0 mmol) of acetone dissolved in 3.0 mL of 1,2-dimethoxyethane was added thereto drop by drop, and this was refluxed for 4 hours and then allowed to cool. Next, water and 10% hydrochloric acid were added to the reaction solution after cooling to acidify the reaction solution, which solution was subjected to toluene extraction. The toluene layer obtained was washed with a saturated aqueous solution of sodium hydrogen carbonate and saturated saline, after which it was dried using anhydrous magnesium sulfate. Next, the solvent was evaporated off under reduced pressure, and the residue was distilled under reduced pressure to yield 4.71 g (41.5% recovery rate) of (4-t-butylbenzoyl)acetone as a distillate at 9 mmHg and 139 to 145°C.

To 1,2-dimethoxyethane (40 mL), sodium hydride (60%, oily, 4.18 g [0.105 mol]) was added, and this was refluxed. To this, a solution of 4.57 g (20.9 mmol) of the (4-t-butylbenzoyl)acetone obtained and 6.03 g (31.3 mmol) of methyl 4-t-butylbenzoate dissolved in 1,2-dimethoxyethane (40 mL) was added drop by drop, and this was refluxed for 3 hours. Subsequently, water was added little by little to the reaction solution to decompose the excess sodium hydride, after which ethyl acetate and 10% aqueous hydrochloric acid were added to the reaction solution to acidify the reaction solution. Next, extraction with ethyl acetate was performed, and the ethyl acetate layer was washed with saturated saline, after which it was dried with anhydrous magnesium sulfate. Next, the solvent was evaporated off under reduced pressure, after which the residue was recrystallized using n-hexane to yield 5.43 g (68.7% recovery rate) of 1,5-di(4-t-butylphenyl)-1,3,5-pentanetrione.

1.00 g (2.64 mmol) of the 1,5-di(4-t-butylphenyl)-1,3,5-pentanetrione obtained was added to and dissolved in 10 ml of concentrate sulfuric acid cooled with ice water. After this was stirred at 0°C for 1 hour, the reaction solution was added to 500 mL of water. Next, sodium carbonate was added to alkalify the reaction solution, after which the crystal was collected by suction filtration and washed with 500 mL of water. At this time, the electroconductivity of the filtrate was 219 µ Scm⁻¹. This was dried at 80 °C under reduced pressure, and the crystal obtained was recrystallized using n-hexane to yield 0.657 g (69.0% recovery rate) of a compound (Example Compound 2).

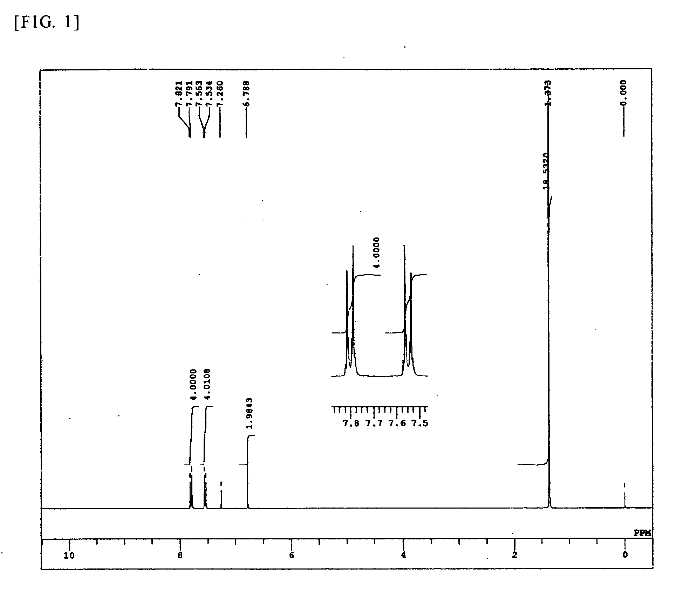

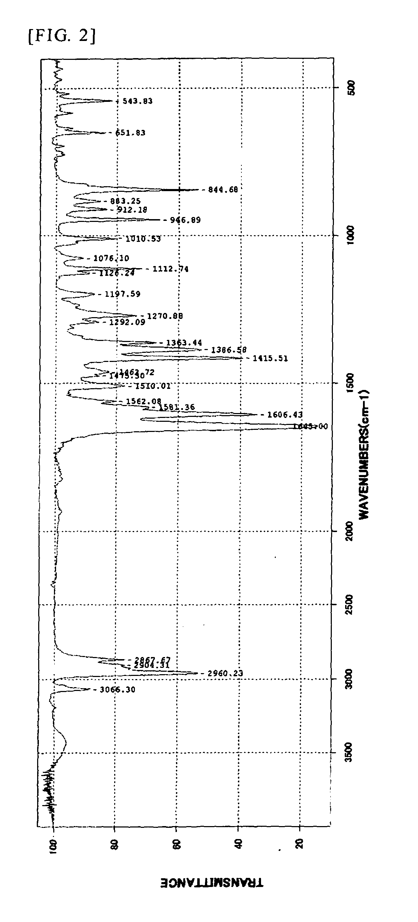

Data on IR, NMR, MS, and elemental analysis of the compound obtained (Example Compound 2) are shown below. Also, a ¹H-NMR spectrum chart and IR spectrum chart of the compound obtained (Example Compound 2) are shown in FIG. 1 and FIG. 2, respectively.

IR (KBr disk) ν (cm⁻¹): 1650 (C=O)
¹H-NMR (CDCl₃) δ (ppm): 1.37 (18H,s,t-Bu)
6.79 (2H,s,=CH-)
7.55 (4H,d,J=8.7Hz,Ar-H)
7.80 (4H,d,J=8.7Hz,Ar-H)
Mass (m/z): 361 (M⁺)
EA (%): Found C, 83.62 H, 8.07
Calculated C, 83.29 H, 7.83 (for C₂₅H₂₈O₂)

Example Compounds 3, 4, and 6 were synthesized in the same manner.

### Example 2 (Synthesis of Example Compound 15)

10.0 g (54.3 mmol) of 4-oxo-4H-pyrane-2,6-dicarboxylic acid was added to 500 mL of THF, and 22.4 g (0.109 mol) of N,N'-dicyclohexylcarbodiimide (DCC) was added while cooling the mixture with ice water. After the mixture was stirred while cooling with ice water for 1 hour, 16.2 g (0.109 mol) of 4-t-butylaniline was added while cooling with ice water. After the mixture was stirred at room temperature for 3 hours, a small amount of water was added to reaction solution, and the precipitated crystal was collected by suction filtration. The crystal obtained was recrystallized using DMF (200 mL), and the crystal was separated by filtration. The filtrate was added to 3 L of water to precipitate a crystal, and this was collected by suction filtration and washed with water. At this time, the electroconductivity of the filtrate was 241 µ Scm⁻¹. The crystal washed was dried, after which it was recrystallized using ethyl acetate to yield 7.69 g (31.7% recovery rate) of a compound (Example Compound 15).

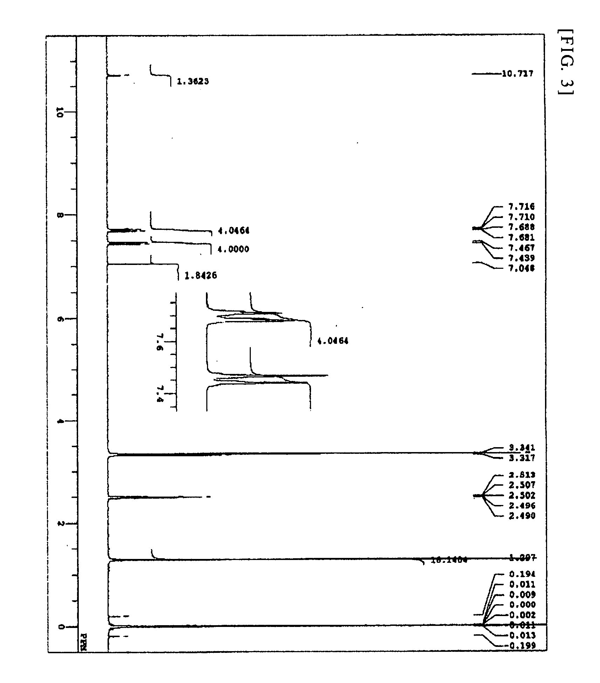

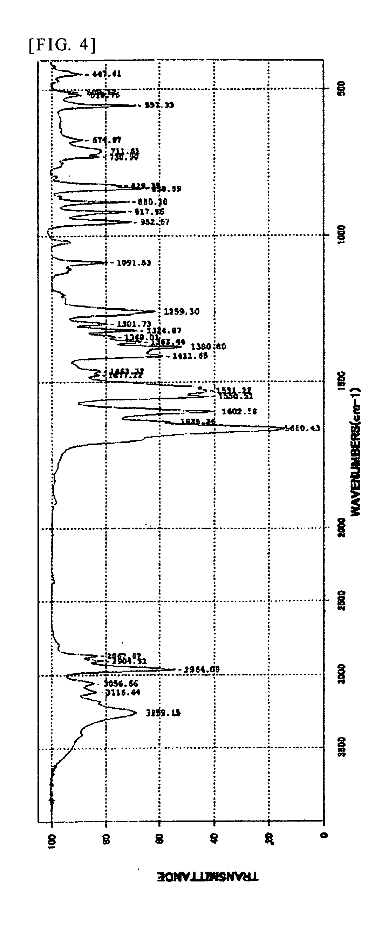

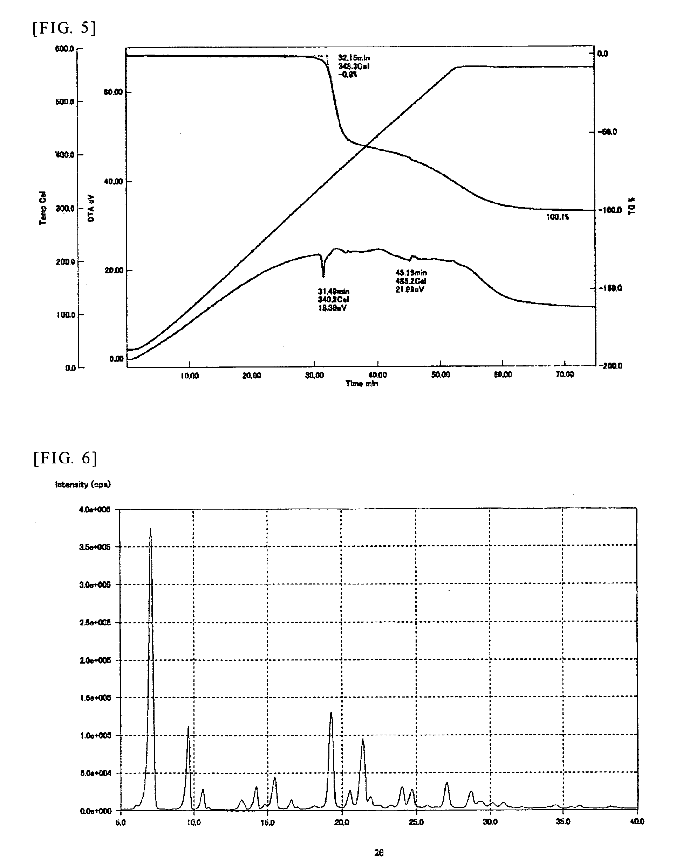

Data on IR, NMR, MS, and elemental analysis of the compound obtained (Example Compound 15) are shown below. Also, a ¹H-NMR spectrum chart, IR spectrum chart, differential thermal/thermogravimetric analysis chart, and X-ray diffraction spectrum chart of the compound obtained (Example Compound 15) are shown in FIG. 3 to FIG. 6, respectively.

IR (KBr disk) ν (cm⁻¹): 3260 (N-H) 1660 (C=O)
¹H-NMR (DMSO-d₆) δ (ppm): 1.30 (18H,s,t-Bu)
7.05 (2H,s,=CH-)
7.45 (4H,d,J=8.4Hz,Ar-H)
7.69 (4H,d,J=8.4Hz,Ar-H)
10.72 (2H,s,N-H)
Mass (m/z): 445 (M⁺-H)
EA (%): Found C, 72.79 H, 7.05 N, 6.32
Calculated C, 72.62 H, 6.77 N, 6.27 (for C₂₇H₃₀N₂O₄)

### Example 3 (Synthesis of Example Compound 17)

5.00 g (24.9 mmol) of 1,4-dihydro-4-oxopyridine-2,6-dicarboxylic acid monohydrate was added to 100 mL of toluene, 14.8 g (0.124 mol) of thionyl chloride and 0.06 mL of DMF were added, and this was refluxed for 6 hours. After this was allowed to cool, the solvent was evaporated off under reduced pressure, 100 mL of THF and 8.82 g (87.2 mmol) of triethylamine were added to the residue, 8.18 g (54.8 mmol) of 4-oxo-4H-pyrane-2,6-dicarboxylic acid was further added, and this mixture was stirred at room temperature for 3 hours. Subsequently, the solvent was concentrated under reduced pressure, and the residue was added to 5 L of water. After this was stirred at room temperature overnight, the crystal was collected by suction filtration, and this was washed with 1000 mL of water. At this time, the electroconductivity of the filtrate was 232 µ Scm⁻¹. After this crystal was dried at 80°C, it was recrystallized from ethyl acetate to yield 4.74 g (42.7% recovery rate) of a compound (Example Compound 17).

Example Compounds 10, 12, 20, 22, 25, and 27 were synthesized in the same manner. Also, Example Compounds 7, 9, 11, 13, 18, 19, 21, 23, 24, 26, 28, 29, and 30 were synthesized in the same manner but the starting material was replaced with 4-oxo-4H-pyrane-2,6-dicarboxylic acid. Note that Example Compound 15 can also be synthesized by this method. Furthermore, Example Compounds 8, 14, and 16 were synthesized by synthesizing diesters in the same manner as Synthesis Example 1, hydrolyzing the ester moieties thereof, and then allowing the reaction to proceed in the same manner. Example Compounds 34 and 35 were synthesized using 4-oxo-4H-pyrane-2,6-dicarboxylic acid as the starting material, and also using the corresponding alcohols in place of amine.

In T.S. Wheeler, "Organic Syntheses" Coll. Vol. IV, p.478 (1963), a method of synthesizing 2-phenyl-4H-1-benzopyran-4-one from 2-hydroxyacetophenone and benzoyl chloride via esterification and a transfer reaction, followed by cyclization, is described. Example Compound 37 was synthesized in the same manner as this method.

In J. R. Prfister, W.E. Wymann, et. al., J. Med. Chem., 23(3), 335-338 (1980), a 6-carboxy-2-phenyl-4H-1-benzopyran-4-one derivative having a substituent in the phenyl group thereof was synthesized via an ethyl 4-acetoxybenzoate transfer reaction, subsequent condensation with an aldehyde derivative, and a cyclizing reaction. Example Compound 39 was synthesized in the same manner as this method. Also, this was amidated in the same manner as Example 2 to yield Example Compound 40.

In Michael P. Sammes, et. al., J. Chem. Soc. Perkin Trans. I, 5, 1585-90 (1981), a ketal was produced from 2,4-pentanedione and ethylene glycol, and subsequently condensed with diethyl oxalate and cyclized to synthesize 6-methyl-4-oxo-4H-pyrane-2-carboxylic acid. Example Compound 32 was synthesized in the same manner as this method. Also, this was amidated in the same manner as Example 2 to yield Example Compound 42.

In M. Yamato and Y. Kusunoki, Chem. Pharm. Bull., 29 (5), 1214-1220 (1981), diethyl acetonedicarboxylate was complexed with magnesium, and the complex was cyclized simultaneously with acylation, whereby 3,5-diethoxycarbonyl-2,6-dimethyl-4H-pyran-4-one was synthesized. Example Compound 43 was obtained by performing a synthesis in the same manner as this method, hydrolyzing the ester moiety, and then performing amidation in the same manner as Example 2.

In M. Stiles and J.P. Selegue, J. Org. Chem., 56, 4067-4070 (1991), a method of synthesizing 4-oxo-6-phenyl-4H-pyrane-2-carboxylic acid by synthesizing a triketo acid from 1-phenylbutane-1,3-dione and dimethyl oxalate, and cyclizing this triketo acid is described. Example Compound 33 was synthesized in the same manner as this method.

In J.S. Bradshaw, P. Huszthy, et. al., Supermolecule Chemistry, Vol.1, 267-275 (1993), a method of dimethyl-esterifying 4-1H-pyridone-2,6-dicarboxylic acid in methanol using thionyl chloride is described. Example Compound 36 was synthesized in the same manner as this method.

In Kumari Sadhana Banerjee and S.S. Deshapande, J. Indian. Chem. Soc., 52(1), 41-44 (1975), 2-ethyl-6-methyl-1,4-dihydro-4-oxopyridine was synthesized by reacting 2-ethyl-6-methyl-4-oxo-4H-pyrane with ammonia in a sealed tube. Example Compounds 5, 38, and 41 were synthesized by using this reaction on pyrone derivatives synthesized in the same manner as Example 1, Example Compound 37, and Example Compound 40, respectively.

### Example 4

Styrene-acrylic copolymer resin [produced by Sanyo Kasei Co., Ltd., product name: CPR-600B]...100 parts
Low polymer polypropylene [produced by Sanyo Kasei Co., Ltd., product name: Biscol 550P]...3 parts
Carbon black [produced by Mitsubishi Chemical Corporation, product name: MA-100]...6 parts
Charge control agent (Example Compound 15)...1 part

The above ingredients were uniformly pre-mixed using a high-speed mill to yield a premix. This premix was kneaded in a molten state using a heat roll, and this kneaded product was cooled and thereafter roughly milled using an ultracentrifugal milling machine. The rough milling product obtained was finely pulverized using an air jet mill equipped with a classifier to yield a black toner having an average particle diameter of 10 µm.
Five parts of the toner obtained was admixed with 95 parts of a ferrite carrier (produced by Powdertech Co., Ltd., product name: F-150) to yield a developer.

This developer was weighed in a polyethylene bottle and stirred in a ball mill at a rotation rate of 100 rpm to charge the developer, and changes over time in charge amount were measured under standard conditions (20°C, relative humidity 60%). The results of determinations of frictional charge amount at different stirring times (minutes) are shown in Table 1. Also, the results of determinations of the amount of initial blowoff charges under low-temperature low-humidity (5°C, relative humidity 30%) conditions and high-temperature high-humidity (35°C, relative humidity 90%) conditions in the same manner (stirring time: 10 minutes), that is, the results of determinations of the environmental stability of charge amount are shown in Table 2.
Note that the determinations of charge amount were performed using a blowoff charge analyzer manufactured by Toshiba Chemical Company [product name: TB-200].

This developer was used to form toner images using a commercial copying machine [a model using an organic photoconductor (OPC) drum]. The toner images obtained were visually examined for fogging, line reproducibility, charge stability and sustainability, and the offset phenomenon, and each parameter was evaluated in two grades or three grades. The results of the evaluation of the toner images are shown in Table 3.

Regarding fogging, toner images with no fogging were given the rating ○, and those with fogging were given the rating ×. Regarding line reproducibility, toner images with good line reproducibility were given the rating ○, those with particularly good line reproducibility were given the rating ⊚, and those with poor line reproducibility were given the rating ×. Regarding charge stability and sustainability, toners with good charge stability and sustainability were given the rating ○, those with particularly good charge stability and sustainability were given the rating ⊚, and those with poor charge stability and sustainability were given the rating ×. Regarding the offset phenomenon, toner images with no offset phenomenon observed were given the rating ○, and those with the offset phenomenon observed were given the rating ×.

### Example 5

Styrene-acrylic copolymer resin [produced by Sanyo Kasei Co., Ltd., product name: CPR-600B]...100 parts
Low polymer polypropylene [produced by Sanyo Kasei Co., Ltd., product name: Biscol 550P]...3 parts
C.I. Pigment Red 122...5 parts
Charge control agent [Example Compound 15]...1 part

The above ingredients were treated in the same manner as Example 4 to yield a magenta toner and a developer; the results of determinations of changes over time in charge amount and the environmental stability of charge amount determined in the same manner as Example 4 are shown in Table 1 and Table 2, respectively.

Also, the results of an evaluation of toner images formed using this developer in the same manner as Example 4 are shown in Table 3.

### Example 6

Styrene-acrylic copolymer resin [produced by Sanyo Kasei Co., Ltd., product name: CPR-600B]...100 parts
Low polymer polypropylene [produced by Sanyo Kasei Co., Ltd., product name: Biscol 550P]...3 parts
C.I. Pigment Yellow 180...5 parts
Charge control agent [Example Compound 15]...1 part

The above ingredients were treated in the same manner as Example 4 to yield a yellow toner and a developer; the results of determinations of changes over time in charge amount and the environmental stability of charge amount determined in the same manner as Example 4 are shown in Table 1 and Table 2, respectively.

Also, the results of an evaluation of toner images formed using this developer in the same manner as Example 4 are shown in Table 3.

### Example 7

Styrene...80 parts
n-butyl methacrylate...20 parts
C.I. Pigment Yellow 180...5 parts
2,2'-azoisobutyronitrile...1.8 parts
Charge control agent (Example Compound 15)...1 part

The above ingredients were uniformly pre-mixed using a high-speed mixer to yield a polymerizable monomer composition.

On the other hand, 100 ml of an aqueous solution of trisodium phosphate at a concentration of 0.1 mol% was diluted with 600 ml of distilled water, and while stirring this liquid, 18.7 ml of an aqueous solution of calcium chloride at a concentration of 1.0 mol/litter was gradually added to this liquid. Furthermore, while stirring this mixed liquid, 0.15 g of an aqueous solution of sodium dodecylbenzenesulfonate at a concentration of 20% by weight was added to this mixed liquid to yield a dispersion.

This dispersion was added to the aforementioned polymerizable monomer composition, and while the mixture was stirred at high speed using the TK Homo-mixer (produced by Tokushu Kika Kogyo), it was heated to a temperature of 65°C ; after the heating, the mixture was stirred for 30 minutes and thereafter further heated to 80°C and stirred at a rotation rate of 100 rpm using an ordinary stirring machine to cause polymerization while maintaining a temperature of 80°C for 6 hours.

After completion of the polymerization, the reaction mixture was cooled, the solid matter was separated by filtration, and filter cake was immersed in an aqueous solution of hydrochloric acid at a concentration of 5% by weight, whereby the calcium phosphate used as the dispersing agent was decomposed. The solid matter obtained was washed with water until neutral washings were obtained, and was dewatered and dried to yield a yellow toner having an average particle diameter of 13 µm.

Five parts of the polymerized toner obtained was admixed with 95 parts of a ferrite carrier (produced by Powdertech Co., Ltd., product name: F-150) to yield a developer; the results of determinations of changes over time in charge amount and the environmental stability of charge amount determined in the same manner as Example 4 are shown in Table 1 and Table 2, respectively.

Also, the results of an evaluation of toner images formed using this developer in the same manner as Example 4 are shown in Table 3.

### Example 8

Styrene...80 parts
n-butyl methacrylate...20 parts
Carbon black [produced by Mitsubishi Chemical Corporation, product name: MA-100]...5 parts
2,2'-azoisobutyronitrile...1.8 parts
Charge control agent (Example Compound 15)...1 part

The above ingredients were treated in the same manner as Example 7 to yield a black polymerized toner and a developer; the results of determinations of changes over time in charge amount and the environmental stability of charge amount determined in the same manner as Example 4 are shown in Table 1 and Table 2, respectively.

Also, the results of an evaluation of toner images formed using this developer in the same manner as Example 4 are shown in Table 3.

### Example 9

Styrene-acrylic copolymer resin [produced by Sanyo Kasei Co., Ltd., product name: CPR-600B]...100 parts
Low polymer polypropylene [produced by Sanyo Kasei Co., Ltd., product name: Biscol 550P]...3 parts
C.I. Pigment Red 122...5 parts

The above ingredients were uniformly pre-mixed using a high-speed mill to yield a premix. This premix was kneaded in a molten state using a heat roll, and this kneaded product was cooled and thereafter roughly milled using an ultracentrifugal milling machine. The rough milling product obtained was finely pulverized using an air jet mill equipped with a classifier to yield mother particles for a magenta toner having an average particle diameter of 10 µm.

One part by weight of offspring particles of Example Compound 15 (charge control agent) were externally added to 108 parts by weight of the mother particles obtained, whereby a toner was obtained.

Five parts by weight of this toner was admixed with 95 parts by weight of a ferrite carrier (F-150: product name, produced by Powdertech Co., Ltd.) to yield a developer.

This developer was weighed in a polyethylene bottle and stirred in a ball mill at a rotation rate of 100 rpm to charge the developer, and changes over time in charge amount were determined. The results of determinations of changes over time in charge amount and the environmental stability of charge amount determined in the same manner as Example 4 are shown in Table 1 and Table 2, respectively.

Also, the results of an evaluation of toner images formed using this developer in the same manner as Example 4 are shown in Table 3.

### Example 10

Polyester resin [produced by Mitsubishi Rayon Co., Ltd., product name: Diacron ER-561]...100 parts
Carbon black [produced by Mitsubishi Chemical Corporation, product name: MA-100]...5 parts
Charge control agent (Example Compound 15)...1 part

The above ingredients were treated in the same manner as Example 4 to yield a black toner and a developer; the results of determinations of changes over time in charge amount and the environmental stability of charge amount determined in the same manner as Example 4 are shown in Table 1 and Table 2, respectively.

Also, the results of an evaluation of toner images formed using this developer in the same manner as Example 4 are shown in Table 3.

### Examples 11 to 25

In Examples 11 to 25, a black toner and a developer were obtained in the same manner as Example 4 except that the charge control agent was replaced with Example Compounds 1, 2, 4, 5, 8, 12, 14, 17, 20, 23, 26, 27, 33, 37, and 43, respectively. The results of determinations of changes over time in charge amount and the environmental stability of charge amount determined in the same manner as Example 4 are shown in Table 1 and Table 2, respectively.

Also, the results of an evaluation of toner images formed using this developer in the same manner as Example 4 are shown in Table 3.

### Comparative Example 1

A black toner and a developer were obtained in the same manner as Example 4 except that the charge control agent was replaced with the Example Compound 1 described in Japanese Patent Laid-Open No. HEI-4-139461 (shown below). The results of determinations of changes over time in charge amount and the environmental stability of charge amount determined in the same manner as Example 4 are shown in Table 1 and Table 2, respectively.

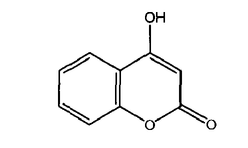

### Comparative Example 2

A black toner and a developer were obtained in the same manner as Example 4 except that the charge control agent was replaced with the Example Compound 2 described in Japanese Patent Laid-Open No. HEI-4-139461 (shown below). The results of determinations of changes over time in charge amount and the environmental stability of charge amount determined in the same manner as Example 4 are shown in Table 1 and Table 2, respectively.

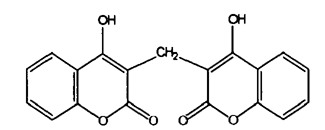

### Comparative Example 3

A black toner and a developer were obtained in the same manner as Example 4 except that the charge control agent was replaced with the Compound 1 described in Japanese Patent Laid-Open No. HEI-8-50372 (shown below). The results of determinations of changes over time in charge amount and the environmental stability of charge amount determined in the same manner as Example 4 are shown in Table 1 and Table 2, respectively.

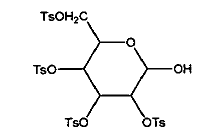

### Comparative Example 4

A black toner and a developer were obtained in the same manner as Example 4 except that the charge control agent was replaced with the Compound 8 described in Japanese Patent Laid-Open No. HEI-8-50372 (shown below). The results of determinations of changes over time in charge amount and the environmental stability of charge amount determined in the same manner as Example 4 are shown in Table 1 and Table 2, respectively.

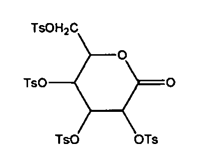

### Comparative Example 5

A black toner and a developer were obtained in the same manner as Example 4 except that the charge control agent was replaced with the Compound shown in Example 1 described in Japanese Patent Laid-Open No. HEI-10-239910 (shown below). The results of determinations of changes over time in charge amount and the environmental stability of charge amount determined in the same manner as Example 4 are shown in Table 1 and Table 2, respectively.

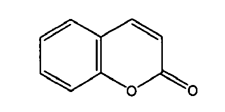

### Comparative Example 6

A black toner and a developer were obtained in the same manner as Example 4 except that the charge control agent was replaced with the compound shown in Example 15 described in Japanese Patent Laid-Open No. HEI-10-239910 (shown below). The results of determinations of changes over time in charge amount and the environmental stability of charge amount determined in the same manner as Example 4 are shown in Table 1 and Table 2, respectively.

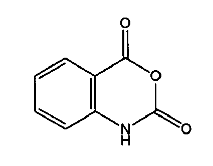

### Comparative Example 7

A black toner and a developer were obtained in the same manner as Example 4 except that the charge control agent was replaced with the compound shown in Example 24 described in Japanese Patent Laid-Open No. HEI-10-239910 (shown below). The results of determinations of changes over time in charge amount and the environmental stability of charge amount determined in the same manner as Example 4 are shown in Table 1 and Table 2, respectively.

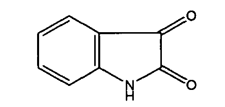

### Comparative Example 8

A black toner and a developer were obtained in the same manner as Example 4 except that the charge control agent was replaced with 2,6-dimethylquinone (shown below). The results of determinations of changes over time in charge amount and the environmental stability of charge amount determined in the same manner as Example 4 are shown in Table 1 and Table 2, respectively.

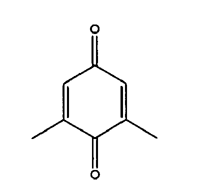

**[Table 1]**

| | | Stirring time (minutes) | | |
|---|---|---|---|---|
| | | 3 | 10 | 30 |
| | Example 4 | -32.4 | -40.1 | -44.3 |
| | Example 5 | -27.6 | -37.4 | -41.7 |
| | Example 6 | -24.7 | -37.4 | -44.8 |
| | Example 7 | -29.7 | -39.0 | -43.8 |
| | Example 8 | -27.9 | -36.8 | -41.6 |
| | Example 9 | -25.7 | -34.3 | -39.4 |
| | Example 10 | -27.1 | -38.4 | -47.3 |
| | Example 11 | -21.7 | -28.7 | -32.6 |
| | Example 12 | -12.4 | -21.5 | -24.6 |
| | Example 13 | -16.2 | -26.9 | -30.1 |
| | Example 14 | -19.7 | -28.0 | -32.2 |
| | Example 15 | -13.8 | -21.7 | -23.5 |
| | Example 16 | -14.5 | -22.4 | -24.7 |
| | Example 17 | -27.8 | -32.7 | -34.1 |
| | Example 18 | -18.3 | -25.2 | -28.7 |
| | Example 19 | -19.6 | -28.3 | -32.8 |
| | Example 20 | -25.4 | -33.1 | -35.9 |
| Charge amount (µC/g) | Example 21 | -26.1 | -35.1 | -38.6 |
| | Example 22 | -13.6 | -23.9 | -28.6 |
| | Example 23 | -14.6 | -22.2 | -25.1 |
| | Example 24 | -23.9 | -30.5 | -34.1 |
| | Example 25 | -20.2 | -25.7 | -28.8 |
| | Comparative Example 1 | -4.5 | -10.2 | -15.2 |
| | Comparative Example2 | -2.8 | -5.7 | -10.3 |
| | Comparative Example 3 | -1.2 | -3.9 | -9.5 |
| | Comparative Example 4 | -1.7 | -5.7 | -9.8 |
| | Comparative Example5 | -2.8 | -6.5 | -14.4 |
| | Comparative Example 6 | -2.5 | -4.3 | -10.0 |
| | Comparative Example 7 | -3.7 | -7.7 | -11.2 |
| | Comparative Example 8 | -3.3 | -6.9 | -14.3 |

**[Table 2]**

| | Charge amount (µC/g) | | |
|---|---|---|---|
| | 5°C-30%RH | 20°C-60%RH | 35°C-90%RH |
| Example 4 | -40.4 | -40.1 | -39.9 |
| Example 5 | -37.6 | -37.4 | -37.3 |
| Example 6 | -37.8 | -37.4 | -36.8 |
| Example 7 | -39.3 | -39.0 | -38.8 |
| Example 8 | -36.8 | -36.8 | -36.5 |
| Example 9 | -35.1 | -34.3 | -33.8 |
| Example 10 | -38.6 | -38.4 | -38.1 |
| Example 11 | -28.8 | -28.7 | -28.3 |
| Example 12 | -21.9 | -21.5 | -21.1 |
| Example 13 | -27.1 | -26.9 | -26.1 |
| Example 14 | -28.7 | -28.0 | -27.3 |
| Example 15 | -22.5 | -21.7 | -21.0 |
| Example 16 | -22.9 | -22.4 | -21.7 |
| Example 17 | -33.1 | -32.7 | -32.2 |
| Example 18 | -26.1 | -25.2 | -24.7 |
| Example 19 | -28.7 | -28.3 | -27.9 |
| Example 20 | -33.3 | -33.1 | -32.2 |
| Example 21 | -35.7 | -35.1 | -34.4 |
| Example 22 | -24.4 | -23.9 | -23.3 |
| Example 23 | -22.3 | -22.2 | -22.0 |
| Example 24 | -30.7 | -30.5 | -30.4 |
| Example 25 | -25.8 | -25.7 | -25.5 |
| Comparative Example 1 | -12.9 | -10.2 | -6.7 |
| Comparative Example 2 | -8.4 | -5.7 | -3.1 |
| Comparative Example 3 | -10.3 | -3.9 | -1.2 |
| Comparative Example 4 | -11.4 | -5.7 | -2.2 |
| Comparative Example 5 | -10.1 | -6.5 | -3.9 |
| Comparative Example 6 | -8.7 | -4.3 | -1.9 |
| Comparative Example 7 | -12.8 | -7.7 | -4.7 |
| Comparative Example 8 | -12.0 | -6.9 | -2.1 |

**[Table 3]**

| Example | Fogging | Line reproducibility | Charge stability and sustainability | Offset phenomenon |
|---|---|---|---|---|
| 4 | ○ | ⊚ | ○ | ○ |
| 5 | ○ | ○ | ○ | ○ |
| 6 | ○ | ○ | ⊚ | ○ |
| 7 | ○ | ⊚ | ⊚ | ○ |
| 8 | ○ | ⊚ | ⊚ | ○ |
| 9 | ○ | ○ | ○ | ○ |
| 10 | ○ | ○ | ⊚ | ○ |
| 11 | ○ | ○ | ○ | ⊚ |
| 12 | ○ | ○ | ○ | ○ |
| 13 | ○ | ○ | ○ | ○ |
| 14 | ⊚ | ○ | ○ | ○ |
| 15 | ○ | ○ | ○ | ○ |
| 16 | ○ | ○ | ⊚ | ○ |
| 17 | ○ | ○ | ○ | ○ |
| 18 | ○ | ⊚ | ○ | ○ |
| 19 | ○ | ○ | ⊚ | ⊚ |
| 20 | ○ | ⊚ | ⊚ | ○ |
| 21 | ○ | ○ | ⊚ | ○ |
| 22 | ○ | ○ | ○ | ○ |
| 23 | ○ | ○ | ○ | ○ |
| 24 | ○ | ⊚ | ○ | ○ |
| 25 | ○ | ⊚ | ○ | ○ |

### BRIEF DESCRIPTION OF THE DRAWINGS

[FIG. 1] A ¹H-NMR spectrum chart of a compound obtained in Example 1 (Example Compound 2).
[FIG. 2] An IR spectrum chart of a compound obtained in Example 1 (Example Compound 2).
[FIG. 3] A ¹H-NMR spectrum chart of a compound obtained in Example 2 (Example Compound 15).
[FIG. 4] An IR spectrum chart of a compound obtained in Example 2 (Example Compound 15).
[FIG. 5] A differential thermal/thermogravimetric analytical chart of a compound obtained in Example 2 (Example Compound 15).
[FIG. 6] An X-ray diffraction spectrum chart of a compound obtained in Example 2 (Example Compound 15).

## Claims

1. A charge control agent having a compound represented by the following General Formula (I) as the active ingredient: 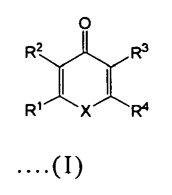 in Formula (I),
X represents an oxygen atom or N-H,
each of R¹ to R⁴ independently represents:
a hydrogen atom,
a carboxyl group,
an aminocarbonyl group having or not having a substituent,
an aminocarbonylmethyl group having or not having a substituent,
an alkoxycarbonyl group,
an alkyl group,
a phenyl group having a substituent or not having a substituent,
a naphthyl group having a substituent or not having a substituent, or
a group that forms a saturated or unsaturated ring having or not having a substituent in cooperation with any other group selected from among R¹ to R⁴.

2. The charge control agent of Claim 1, wherein the compound of General Formula (I) above is 1 or 2 or more selected from among the following compounds 1 to 6:
Compound 1 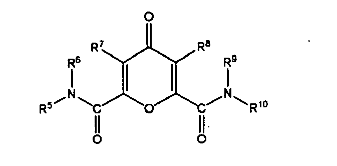
Compound 2 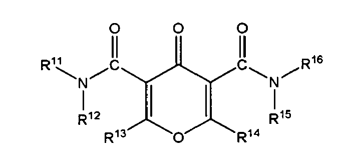
Compound 3 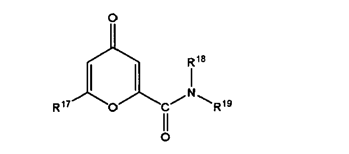
Compound 4 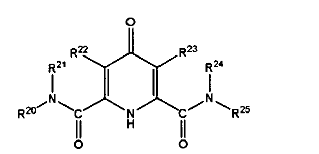
Compound 5 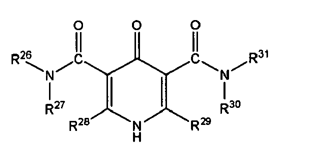
Compound 6 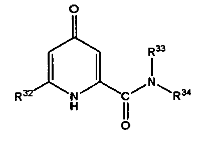
in Compounds 1 to 6,
each of R⁵ to R³⁴ independently represents a hydrogen atom, an alkyl group, a cycloalkyl group not having a substituent or having a substituent, a phenyl group having a substituent or not having a substituent, or a naphthyl group having a substituent or not having a substituent,
each of the aforementioned phenyl group having a substituent and the naphthyl group having a substituent has 1 or 2 or more selected from among alkyl groups, cycloalkyl groups, halogen atoms, sulfonic acid ester groups, aryl groups, acylamino groups, arylcarbonylamino groups, aminocarbonyl groups, alkoxy groups, acyl groups, or arylcarbonyl groups as substituents on the aromatic ring thereof; provided that 2 or more substituents are present, they may be mutually identical or different.

3. The charge control agent of Claim 1, wherein the compound of General Formula (I) above is one or both of the following compounds 7 and 8:
Compound 7 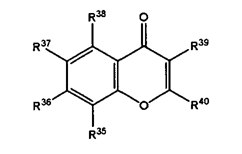
Compound 8 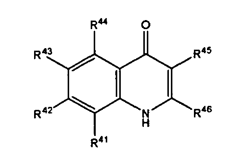 in Compounds 7 and 8,
each of R³⁵ to R⁴⁶ independently represents a hydrogen atom, an alkyl group, a phenyl group having a substituent or not having a substituent, a naphthyl group having a substituent or not having a substituent, an aminocarbonyl group having or not having a substituent, or a carboxyl group,
each of the aforementioned phenyl group having a substituent and the naphthyl group having a substituent has 1 or 2 or more selected from among alkyl groups, cycloalkyl groups, halogen atoms, sulfonic acid ester groups, aryl groups, acylamino groups, arylcarbonylamino groups, aminocarbonyl groups, alkoxy groups, acyl groups, or arylcarbonyl groups as substituents on the aromatic ring thereof; provided that 2 or more substituents are present, they may be mutually identical or different.

4. The charge control agent of Claim 1, wherein the compound of General Formula (I) above is one or both of the following Compounds 9 and 10:
Compound 9 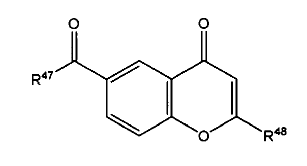
Compound 10 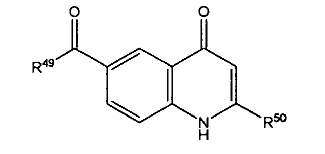 in Compounds 9 and 10,
each of R⁴⁷ and R⁴⁹ independently represents a hydroxyl group or an amino group having a substituent or not having a substituent,
each of R⁴⁸ and R⁵⁰ independently represents a hydrogen atom, an alkyl group, a phenyl group having a substituent or not having a substituent, or a naphthyl group having a substituent or not having a substituent,
each of the aforementioned phenyl group having a substituent and the naphthyl group having a substituent has 1 or 2 or more selected from among alkyl groups, cycloalkyl groups, halogen atoms, sulfonic acid ester groups, aryl groups, acylamino groups, arylcarbonylamino groups, aminocarbonyl groups, alkoxy groups, acyl groups, or arylcarbonyl groups as substituents on the aromatic ring thereof; provided that 2 or more substituents are present, they may be mutually identical or different.

5. A toner for developing electrostatic images, which comprises a resin for toner, a coloring agent, and the charge control agent of any one of Claims 1 to 4.

6. A charge control method comprising controlling the charge of a resin powder by containing the charge control agent of any one of Claims 1 to 4 in the resin powder.

7. A compound represented by the following Formula (II): 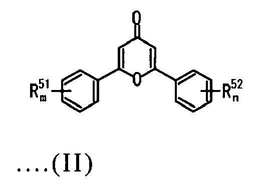 in Formula (II),
each of R⁵¹ and R⁵² independently represents an unbranched or branched alkyl group having 1 to 18 carbon atoms,
each of m and n independently represents an integer of 0 to 3.

8. A compound represented by the following Formula (III): 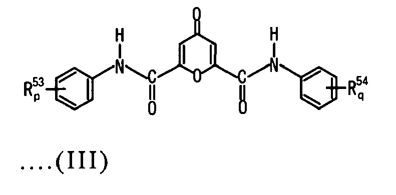 in Formula (III),
each of R⁵³ and R⁵⁴ independently represents an unbranched or branched alkyl group having 1 to 18 carbon atoms,
each of p and q independently represents an integer of 0 to 3.
